# EUROPÄISCHE PATENTSCHRIFT

(11) **EP 1 524 983 B1**
(45) Veröffentlichungstag und Bekanntmachung des Hinweises auf die Patenterteilung: **02.01.2008**
(21) Anmeldenummer: 03771100.9
(22) Anmeldetag: 25.07.2003
(51) Int. Cl.: A61K 31/565, A61P 15/16

(54) **ZUSAMMENSETZUNG, ENTHALTEND EIN ANDROGENES 11-B-HALOGENSTEROID UND EIN GESTAGEN SOWIE M NNLICHES KONTRAZEPTIVUM AUF BASIS DIESER ZUSAMMENSETZUNG**
COMPOSITION CONTAINING AN ANDROGENOUS 11-B-HALOGEN STEROID AND A PROGESTATIONAL HORMONE, AND MALE CONTRACEPTIVE BASED ON SAID COMPOSITION
COMPOSITION CONTENANT UN 11-B-HALOGENOSTEROIDE ANDROGENE ET UN GESTAGENE, ET CONTRACEPTIF MASCULIN A BASE DE CETTE COMPOSITION

(30) Priorität: 25.07.2002 DE 10234525; 25.02.2003 US 449400 P
(43) Veröffentlichungstag der Anmeldung: 27.04.2005
(73) Patentinhaber: Bayer Schering Pharma Aktiengesellschaft, 13353 Berlin (DE)
(72) Erfinder: NUBBEMEYER, Reinhard, 13467 Berlin (DE); HABENICHT, Ursula-Friederike, 14167 Berlin (DE); BOHLMANN, Rolf, 14055 Berlin (DE)
(86) Internationale Anmeldenummer: PCT/EP2003/008192
(87) Internationale Veröffentlichungsnummer: WO 2004/011008

(56) Entgegenhaltungen:
- WO-A-00/21570
- WO-A-01/60376
- WO-A-02/49622
- WO-A-02/059139
- DE-A- 19 650 352
- US-A- 4 925 834
- US-A- 5 952 319

## Beschreibung

Die vorliegende Erfindung betrifft im weiteren Sinne eine Zusammensetzung, enthaltend ein androgenes 11 β-Halogensteroid, ausgewählt aus der Gruppe der Verbindungen der allgemeinen Formel I 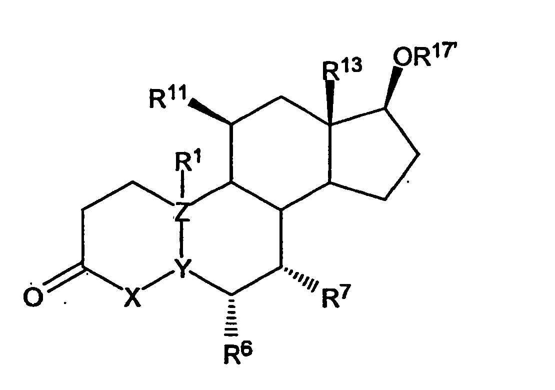 worin
X-Y-Z eine Gruppe mit einer der beiden Strukturen CH=C-C oder CH₂-C=C darstellt,
R¹ α- und β-ständig sein kann und für Wasserstoff, R oder über P an die Ringgrundstruktur gebundenes P-Q-R steht, wobei P und Q gerad- oder verzweigtkettige C₁- bis C₈-Alkylen-, -Alkenylen-,
-Alkinylengruppen oder deren fluorierte Derivate darstellen und gleich oder verschieden sein können und wobei R einen CH₃- oder CF₃-Rest darstellt, mit der Maßgabe, daß an Z kein Substituent R¹ vorhanden ist, wenn X-Y-Z die Gruppe CH₂C=C darstellt,
R⁶ ein Wasserstoffatom ist oder die unter R⁷ angegebenen Bedeutungen haben kann,
R⁷ für R oder über P an die Ringgrundsstruktur gebundenes P-Q-R steht, wobei diese Gruppen die vorerwähnten Bedeutungen haben,
R¹¹ ein Halogen darstellt,
R¹³ Methyl oder Ethyl ist und
R^{17'} Wasserstoff ist oder für C(O)-R¹⁸ steht, wobei
R¹⁸ ein gerad- oder verzweigtkettiger C₁- bis C₁₈-Alkyl-, -Alkenyl-, - Alkinylrest oder ein Arylrest ist, oder für über P an die C(O)-Gruppe gebundenes T-U-V steht, wobei T und U gerad- oder verzweigtkettige C₁- bis C₁₈-Alkylen-, -Alkenylen-, -Alkinylengruppen, alicyclische C₃- bis C₁₂-Gruppen oder Arylgruppen darstellen und gleich oder verschieden sind, und V ein gerad- oder verzweigtkettiger C₁- bis C₁₈-Alkyl-, - Alkenyl- oder -Alkinyl- oder ein Arylrest ist oder
R¹⁸ eine der vorerwähnten Bedeutungen hat und zusätzlich mit einer oder mehreren Gruppen NR¹⁹R²⁰ oder einer oder mehreren Gruppen SOₓR²¹ substituiert ist, wobei x = 0,1 oder 2 und R¹⁹, R²⁰ und R²¹ jeweils Wasserstoff oder über T an N, S gebundenes T-U-V mit der vorerwähnten Bedeutung sind, mit der Maßgabe, daß außerdem die physiologisch verträglichen Additionssalze mit anorganischen und organischen Säuren einbezogen sind,
und das Gestagen der nachstehenden Formel.

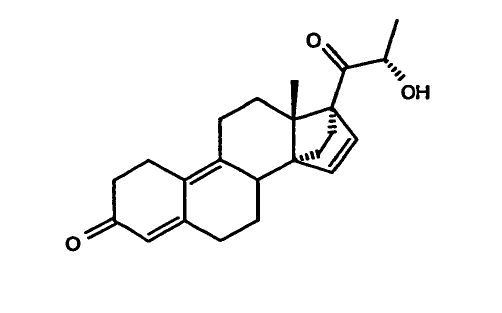

Diese Zusammensetzung ist zur Herstellung von pharmazeutischen Zusammensetzungen geeignet. Deshalb betrifft die vorliegende Erfindung auch pharmazeutische Zusammensetzungen, die eine vorstehend genannte Zusammensetzung aus einem androgenen 11 β-Halogensteroid und dem Gestagen der Formel 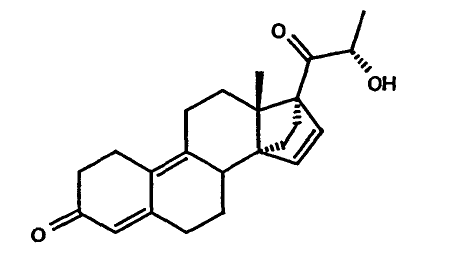 sowie einen pharmakologisch verträglichen Träger und/oder Hilfsstoffe enthalten.

Sowohl in der Zusammensetzung als auch in der pharmazeutischen Zusammensetzung ist als androgenes 11 β-Halogensteroid 11 β-Fluor-17β-hydroxy-7α-methyl-estr-4-en-3-on bevorzugt.

In einer weiteren Ausführungsform betrifft die vorliegende Erfindung ein männliches Kontrazeptivum auf Basis der vorstehenden pharmazeutischen Zusammensetzung. Gemäß einer weiteren Ausführungsform der Erfindung ist in dem männlichen Kontrazeptivum als androgenes 11 β-Halogensteroid 11 β-Fluor-17β-hydroxy-7α-methyl-estr-4-en-3-on enthalten.

In einer besonderen Ausführungsform der vorliegenden Erfindung sind in dem männlichen Kontrazeptivum sowohl das androgene 11 β-Halogensteroid als auch das Gestagen so formuliert, daß beide in Form eines gemeinsamen Implantats oder zweier getrennter Implantate in den Körper des männlichen Anwenders eingesetzt werden können, damit die aktiven Verbindungen über einen längeren Zeitraum an den Organismus des Anwenders abgegeben werden.
Eine kontinuierliche Freisetzung des Gestagens über einen längeren Zeitraum kann auch mit einem transdermalen System, in welches das Gestagen eingebettet ist, bewerkstelligt werden.

Es ist erfindungsgemäß aber auch denkbar, einen der Wirkstoffe in einer oralen Formulierung und den anderen Wirkstoff als Implantat oder transdermal zu verabreichen. Es ist auch möglich, beide Wirkstoffe oral zu verabreichen.
Schließlich sei noch die Möglichkeit erwähnt, eine oder beide der Komponenten der erfindungsgemäßen Zusammensetzung buccal oder transmuccosal zu applizieren.

Das Konzept für die Fertilitätskontrolle beim Mann steht im Einklang mit den von der WHO definierten globalen Zielen zur "Reproduktiven Gesundheit" [Reproductive Health, siehe WHO Task Force on Methods for the Regulation of Male Fertility (1990) Contraceptive efficacy of testosterone-induced azoospermia in normal men; Lancet 336: 955-959; WHO Task Force on Methods for the Regulation of Male Fertility (1993) Comparison of two androgens plus depot-medroxyprogesterone acetate for suppression to azoospermia in Indonesian men; Fertil Steril 60: 1062; WHO Task Force on Methods for the Regulation of Male Fertility (1995) Rate of testosterone-induced suppression to severe oligozoospermia or azoospermia in two multinational clinical studies; Int J Androl 18: 157-165; WHO Task Force on Methods for the Regulation of Male Fertility (1996) Contraceptive efficacy of testosterone-induced azoospermia and oligozoospermia in normal men; Fertil Steril 65; 821-829]. Integraler Bestandteil dieser Strategie sind Kontrazeptiva für Mann und Frau. Da insbesondere kontrazeptive Methoden für den Mann noch fehlen, wird die Entwicklung solcher als unbedingt notwendig angesehen (Andrologie, Grundlagen und Klinik der reproduktiven Gesundheit des Mannes; Hrsg. E. Nieschlag, H.M. Behre; Springer Verlag, 2. Auflage, S. 442 ff, 2000). Am weitesten fortgeschritten in der Entwicklung sind hormonale Ansätze zur männlichen Fertilitätskontrolle. Sie zeichnen sich durch erwiesene Reversibilität und Wirksamkeit aus.
Die hormonale männliche Kontrazeption beruht auf der Suppression (dem Stop) der Spermatogenese, was letztendlich zu einer Azoospermie und somit zur Infertilität des Mannes führt. Mechanistisch werden die beiden Gonadotropine LH (Luteinisierendes Hormon) und FSH (Follikel-stimulierendes Hormon) signifikant gehemmt, d. h. die Serumkonzentrationen dieser beiden Hormone sind nicht mehr detektierbar. Als Folge der LH-Suppression wird die testikuläre Testosteronproduktion ebenfalls gehemmt (beide Hormone gehören zu einem endokrinen Regelkreis). Der Ausfall aller drei Hormone ist zur Hemmung der Spermatogenese notwendig. Der wesentliche Nachteil der beschriebenen Methode ist das Androgendefizit und die daraus resultierenden Symptome/Folgen für den Mann.

Methoden zur männlichen Kontrazeption versuchen LH, FSH und intratestikuläres Testosteron zu supprimieren und damit die Spermatogenese zu unterbinden, während peripheres Testosteron durch ein exogen zugeführtes Androgen substituiert wird. Als Androgen wurde bisher Testosteron bzw. Testosteronester (z. B. Testosteronenanthat, Testosteronbuciclat) verwendet. Die Aufgabe des endokrinen Testosterons besteht in der Aufrechterhaltung der Libido, der Potenz, des männlichen Verhaltens, des Proteinmetabolismus, der Erythropoese und anderer Funktionen, wie Mineral- und Knochenstoffwechsel.
Kurzum, das Ziel besteht darin, das Testosteron in den Testes auf ein Niveau, wie es im peripheren Blut angetroffen wird, abzusenken, während die Spiegel in der allgemeinen Zirkulation aufrecht zu erhalten sind.

Die Suppression der Spermatogenese durch Testosteron bzw. Testosteronester alleine, was im ersten Moment als ein ideales Kontrazeptivum erschien, erwies sich jedoch bisher als nicht effizient genug und zeigte z. T. eine zu lange Zeit bis zum sicheren Wirkeintritt (Onset von bis zu 6 Monaten). Zudem wurden auch ethnische Unterschiede festgestellt. Zu hohe Dosen zeigten deutliche und unerwünschte Nebenwirkungen.
Bei einer Behandlung mit Testosteron hat sich gezeigt, daß sich Nebenwirkungen einstellen, insbesondere eine Vergrößerung der Prostata durch numerische Zunahme der Zellen und Drüsen des Stromas (BPH: benigne Prostatahyperplasie). Bei dem durch 5a-Reduktase vermittelten Metabolismus von Testosteron entsteht Dihydrotestosteron (DHT), das unter anderem zum Auftreten der BPH führen kann (Cummings et al., ibid.; WO 99/13883 A1).
Testosteron ist derzeit nicht oral verfügbar, daher müssten alternative Darreichungsformen (i.m., Pflaster, etc.) angewendet werden.
Um sowohl den Wirkeintritt zu beschleunigen als auch die Effizienz zu verbessern, wurde Testosteron mit anderen Gonadotropin-supprimierenden Substanzen, mit GnRH-Antagonisten (GnRH = Gonadotropin Releasing Hormone) kombiniert. Die Azoospermierate wie auch der Zeitpunkt des Wirkeintritts wurden mit dieser Kombination deutlich verbessert. Allerdings müssen die zur Zeit verfügbaren GnRH-Antagonisten täglich appliziert werden (i.m. oder s.c., die orale Applikation steht nicht zur Verfügung) und ihre Herstellung ist teuer. Daher ist diese Kombination nicht attraktiv.

Die Verwendung entweder der Progestine Cyproteronacetat oder Levonorgestrel war entweder unwirksam in der Unterdrückung der Spermatogenese oder führte in höheren Dosierungen zu einem signifikanten Abfall der Anzahl der roten Blutkörperchen (Merrigiola et al., 1998; Merrigiola et al., 1997; Merrigiola et al., 1996; Bebb et al., 1996).

Die Verwendung einer Mischung zweier Verbindungen, eines Estrogens mit einem Estrogen, in Kombination ist in US 4,210,644 beschrieben.

Eine Methode, die auf die Inhibierung der Spermatogenese durch die perkutane oder orale Gabe von Testosteron und die orale Gabe von Norethisteronacetat abzielt, wurde ebenfalls beschrieben (Guerin und Rollet; 1998). Zur Erreichung einer Azospermie werden allerdings ziemlich hohe Dosen beider Komponenten benötigt.

Zum Ersatz des Testosterons für die männliche Kontrazeption wurde 7α-Methyl-19-nortestosteron (MeNT) vorgeschlagen, das zum einen eine höhere biologische Wirksamkeit als Testosteron aufweist, da es eine höhere Bindungsaffinität zu den Androgenrezeptoren hat Zum anderen widersteht es wegen einer sterischen Hinderung durch die 7a-Methylgruppe vermutlich der Metabolisierung durch 5α-Reduktase (Cummings et al., ibid., WO 99/13883 A1, WO 99/13812 A1, US-A-5,342,834).
Aufgrund der letztgenannten Eigenschaft wird ein deutlich günstigeres Nebenwirkungsprofil im Vergleich zu Testosteron erwartet, speziell im Hinblick auf die Prostata.
Eine Kombination des 7α-Methyl-19-nortestosterons mit einem Gestagen ist diesen Fundstellen nicht zu entnehmen.

Weitere, dem 7a-Methyl-19-nortestosteron in ihrer selektiven Androgenwirkung vergleichbare Verbindungen, sind die erfindungsgemäß zu verwendenden androgenen 11β-Halogensteroide der allgemeinen Formel 1, insbesondere das 11β-Fluor-17β-hydroxy-7α-methyl-estr-4-en-3-on.
Diese Verbindungen sind erstmals in der DE 101 04 327.9 beschrieben. Die Verbindungen verfügen über eine verbesserte metabolische Stabilität gegenüber dem 7α-Methyl-19-nortestosteron. Die DE 101 04 327.9 ist ein nichtvorveröffentlichtes Dokument.
Zur Beschreibung und Definition der Substituenten der Verbindungen der allgemeinen Formel 1 wird auf dieses Dokument verwiesen.

Die Verbindungen sind zur Anwendung in der männlichen Kontrazeption vorgeschlagen. Sie können zusammen mit Gestagenen verwendet werden, ohne daß genauer gesagt wird, um welche Gestagene es sich dabei handeln soll.

Aufgabe der vorliegenden Erfindung ist es, ein männliches Kontrazeptivum auf Androgen-/Gestagen-Basis zur Verfügung zu stellen, welches nicht auf Testosteron als Androgen zurückgreift. Gleichzeitig soll durch das Gestagen die Dosis des zu verwendenden Androgens minimiert und dadurch Nebenwirkungen reduziert werden.

Diese Aufgabe wird durch die eingangs erwähnte Kombination eines androgenen 11 β-Halogensteroids, insbesondere 11β-Fluor-17β-hydroxy-7α-methyl-estr-4-en-3-on, mit dem Gestagen der Formel 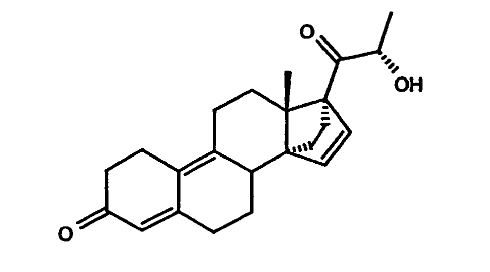 gelöst.

Dieses Gestagen ist in der internationalen Patentanmeldung WO 96/20209 (DE 44 47 401.6) beschrieben. Die gemeinsame Verabreichung mit einem Androgen zur Erzielung einer männlichen Infertilität ist dieser Anmeldung nicht zu entnehmen.
Es handelt sich um ein nach oraler Applikation stark wirksames Gestagen. Aber auch andere Verabreichungsrouten wurden in dieser Anmeldung vorgeschlagen. Des Weiteren ist ein Transdermalsystem, enthaltend dieses Gestagen, in der Patentanmeldung EP 00250449.6 beschrieben.

Mit der Anwendung der vorstehend genannten Kombination als männliches Kontrazeptivum läßt sich eine ausreichende Hemmung der Spermienproduktion im Hoden bei gleichzeitig relativ niedriger Substitutionsdosis des Androgens erreichen. Es wird hierbei ein synergistischer Effekt erreicht.

Mittels der erfindungsgemäßen Zusammensetzung als männlichem Kontrazeptivum gelingt es, mit niedrigen Dosierungen beider Komponenten, die Parameter LH, FSH und Testosteron in den Nichtnachweisbereich bzw. den nicht mehr wirksamen Bereich zu drücken. Der Abfall der Parameter LH und FSH geht miteinander einher.

Für die Zuverlässigkeit und die Akzeptanz des erfindungsgemäßen Kontrazeptivums durch den Mann ist es dabei von entscheidender Bedeutung, daß der Abfall dieser für die Sicherheit des Kontrazeptivums entscheidenden Parameter relativ schnell gelingt. Der "onset" für das erfindungsgemäße Kontrazeptivum liegt ca. 3 Monate nach Beginn der Anwendung.

Die Anwendungsdauer des erfindungsgemäßen Kontrazeptivums kann prinzipiell und gegebenenfalls unbegrenzt sein, d. h. bis vom Anwender keine Kontrazeption mehr benötigt wird.
Andererseits gewährt das erfindungsgemäße Kontrazeptivum jederzeit eine Wiedererlangung der Fertilität des Anwenders.

Die Dosierungen des androgenen 11 β-Halogensteroids der allgemeinen Formel I, insbesondere 11β-Fluor-17β-hydroxy-7α-methyl-estr-4-en-3-on und des Gestagens werden so gewählt, daß die Spiegel von LH, Testosteron und FSH spätestens 3 Monate nach Beginn der Anwendung im nicht mehr wirksamen Bereich dieser Parameter liegen.
Für 11β-Fluor-17β-hydroxy-7α-methyl-estr-4-en-3-on ist eine täglich wirksame Menge von 0,7µg bis 1,5µg, vorzugsweise von 0,7µg bis 1,0µg, ausreichend.
Bei der Bestimmung einer wirksamen Menge des androgenen Steroids der Formel I kann berücksichtigt werden, daß 11 β-Fluor17β-hydroxy-7α-methyl-estr-4-en-3-on etwa 10fach stärker wirksam ist, als Testosteron.
Im Falle der Applikation mittels eines Implantats oder eines anderen, den Wirkstoff über einen längeren Zeitraum abgebenden Systems, muß dieses so beschaffen sein, daß die angegebene Menge täglich freigesetzt wird.

Als Richtschnur für die Dosierung des erfindungsgemäß zu verwendenden Gestagens kann gelten, daß die gewählte Menge auf die Hemmung der Spermatogenese eine vergleichbare Wirkung wie eine tägliche Dosis von 200µg bis 300µg Levonorgestrel hat. Eine einer täglichen oralen Gabe von 240µg bis 260µg Levonorgestrel equieffektiven Menge ist bevorzugt.

Die Ermittlung equieffektiver Mengen von Levonorgestrel und des erfindungsgemäß zu verwendenden Gestagens erfolgt nach dem Fachmann bekannten Methoden, beispielsweise im Schwangerschaftserhaltungstest an der Ratte.

Zur Formulierung der beiden Wirkstoffe im erfindungsgemäßen Kontrazeptivum wird auf die vorstehend genannten Fundstellen, in denen die Wirkstoffe selbst beschrieben sind, verwiesen. Techniken für die Formulierung von Androgenen bzw. Gestagenen zur langanhaltenden Freigabe dieser Wirkstoffe sind im Stand der Technik bekannt, so z. B. die Implant-Systeme Norplant oder Jardelle für Gestagene. Weiterhin wird zur Formulierung des erfindungsgemäß zu verwendenden Gestagens auf die WO 00/21570 (Formulierung mit einem Cyclodextrin) und WO 02/49622 (Transdermalsystem enthaltend das erfindungsgemäß zu verwendende Gestagen) hingewiesen.

Die Bestimmung der Parameter LH, FSH sowie Testosteron erfolgt nach bekannten Methoden.

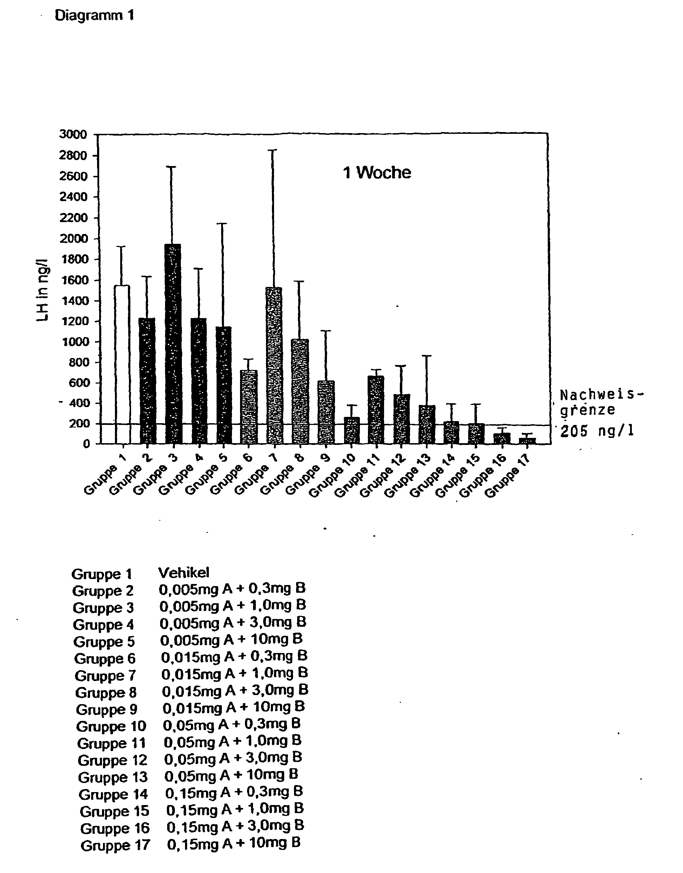

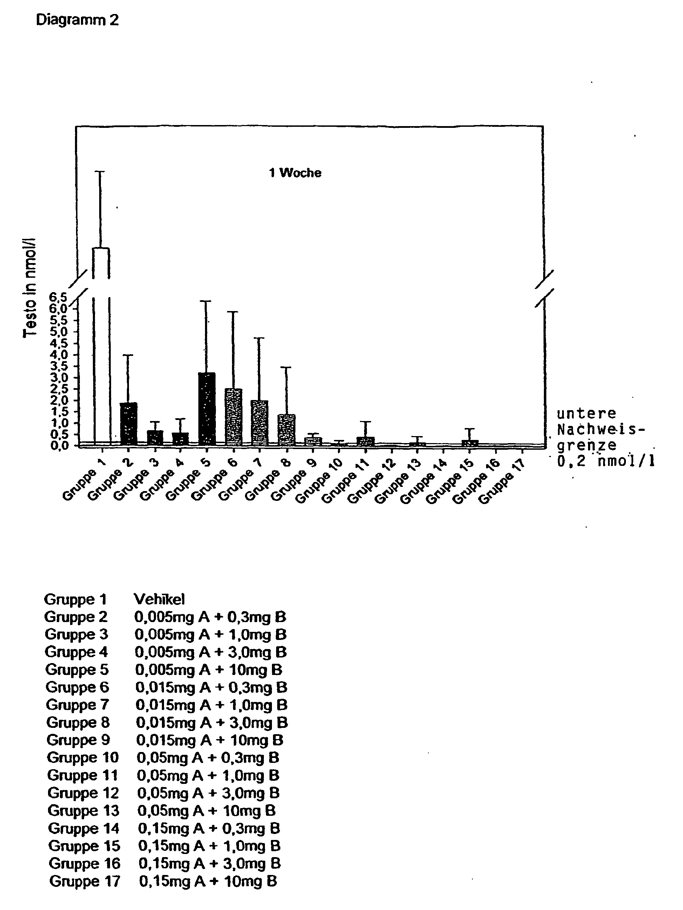

Die Wirksamkeit der erfindungsgemäßen Kombination wird durch
- die Bestimmung der LH-Konzentration im Serum bei der männlichen juvenilen Ratte nach einem Behandlungszeitraum von 1 Woche bei s.c. Applikation einer Kombination der Verbindungen A und B (Diagramm 1) sowie
- die Bestimmung der Testosteron-Konzentration im Serum bei der männlichen adulten Ratte nach einem Behandlungszeitraum von 1 Woche bei s.c. Applikation einer Kombination der Verbindungen A und B (Diagramm 2)
bestätigt.
In beiden Fällen liegen diese Parameter bereits nach einer Woche unterhalb der Nachweisgrenze (schneller Wirkeintritt).
Verbindung A ist 11β-Fluor-17β-hydroxy-7α-methyl-estr-4-en-3-on und Verbindung B ist das erfindungsgemäß zu verwendende Gestagen.
Die angegebenen Dosen wurden pro kg Körpergewicht und täglich verabreicht.

In weiteren Versuchen an der männlichen Ratte konnte gezeigt werden, daß mit der erfindungsgemäßen Kombination (Verbindung A mit Verbindung B; identische Dosierungen wie bei Diagramm 1 und 2 angegeben) nach/bei sechswöchiger Behandlungsdauer
- die relevanten Organgewichte (Prostata, Nebenhoden, Samenblase und Hoden) je nach Hormonstatus gesenkt werden;
- die Spermienzahl auf weniger als 10% des Kontrollwertes gesenkt wird;
- die Werte der Hormone LH und Testosteron auch nach einer über diesen Zeitraum stattfindenden Behandlung stets unter der Nachweisgrenze bleiben (d.h. also sowohl schneller Wirkeintritt, s. o., als auch anhaltende Wirkung).

### Literaturstellen:

- E. Nieschlag and H_M_ Behre; Testosterone in Male Contraception. In E. Nieschlag and H. M. Behre, eds. Testosterone: action, deficiency, substitution, 1998, Springer, Berlin, pp 513-528.
- M.C_ Merrigiola, W.J. Bremner. A. Constantino, A. Pavani, M. Capelli and C. Flamigni; Low Dose of Cyproterone Acetate and Testosterone Enanthate for Contraception in Men_, Hum Reprod., (1998) 13, 1225-1229*.*
- M:C. Merrigiola, W.J. Bremner, A. Constantino, A. Pavant, M. Capelli and C. Flamigni, An Oral Regimen of Cyproterone Acetate and Testosterone Undecanoate for Spermatogenic Suppression in Men, Ferld. Steril. (1997). 68, 84-850*.*
- M.C. Merrigiola, W.J_ Bremner, C.A. Paulsen, A. Valdiserri, L. Incorvaia, R. Motta, A. Pavani, M. Capelli and C. Flamigni, A Combined Regimen of Cyproterone Acetate and Testosterone Enanthate as a Potentially Highly effective Male Contraceptive, J. Clin. Endocrinol. (1996). 81, 3018-3023
- R.A. Bebb, B.D. Anawalt, R.B. Christensen, C.A. Paulsen, W.J. Bremner and A.M. Matsumoto., Combined Administration of Levonorgestrel and Testosterone Induces More Rapid and Effective Suppression of Spermatogenesis than Testosterone AloneA Promising Contraceptive Approach., J. Clin Endocriniol. Metab_(1996) 81, 757-762*.*
- J.F. Guenn and J. Rollet, International Joumal of Andrology, 1988, 11, pp. 187-199*.*
- Hadgraft and Guy; Transdermal Drug Delivery; Developmental Issues and ResearchInitiatives, Marcel Dekker Inc., 1989*.*
- M. Ottel and E. Schillinger (editors), Handbook of Experimental Pharmacology, Vol. 135/11, Androgens and Antiestrogens /1, Pharmacology and Clinical Applications of Estrogens and Antiestrogens; K_-H. Fritzemeier and C. Hegele-Hartung. In Vitro and In Vivo Models to Characterise Estrogens and Antiestrogens; Springer-Verlag, Berlin. Heidelberg, 1999.
- F. Neuman, F. Daher, J. Brotherton, K.-J. GrAf, S.H. Hasan, H.J. Horn, A. Hughes, G.W. Oertel, H. Steinheck, H.E. Voss, R.K. Wagner, Androgens // and AntiAndrogens, Springer-Verlag, Berlin, Heidelberg, 1974.
- Fuhrmann, Bengston. Repenthin, and Schillinger, J. SteroidBiochem. Mol. Biol., 1992, 42(8), 787).
- Lancet 336; 1990, 955-959
- Fertil Steril 60; 1993, 1062
- Int J Androl 18: 1995; 157-165
- Fertil Steril 65; 1996, 821-829
- Andrologie, Grundlagen und Klinik der reproduktiven Gesundheit des Mannes; Hrsg. E. Nieschlag, H.M. Behre; Springer Verlag, 2. Auflage, S. 442 ff, 2000.

## Patentansprüche

1. Zusammensetzung, enthaltend ein androgenes 11 β-Halogensteroid, ausgewählt aus der Gruppe der Verbindungen der allgemeinen Formel I 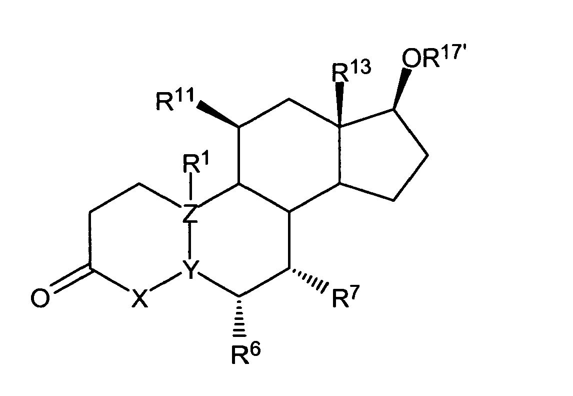 worin
X-Y-Z eine Gruppe mit einer der beiden Strukturen CH=C-C oder CH₂-C=C darstellt,
R¹ α- und β-ständig sein kann und für Wasserstoff, R oder über P an die Ringgrundstruktur gebundenes P-Q-R steht, wobei P und Q gerad- oder verzweigtkettige C₁- bis C₈-Alkylen-, -Alkenylen-,
-Alkinylengruppen oder deren fluorierte Derivate darstellen und gleich oder verschieden sein können und wobei R einen CH₃- oder CF₃-Rest darstellt, mit der Maßgabe, daß an Z kein Substituent R¹ vorhanden ist, wenn X-Y-Z die Gruppe CH₂-C=C darstellt,
R⁶ ein Wasserstoffatom ist oder die unter R⁷ angegebenen Bedeutungen haben kann,
R⁷ für R oder über P an die Ringgrundsstruktur gebundenes P-Q-R steht, wobei diese Gruppen die vorerwähnten Bedeutungen haben,
R¹¹ ein Halogen darstellt,
R¹³ Methyl oder Ethyl ist und
R^{17'} Wasserstoff ist oder für C(O)-R¹⁸ steht, wobei
R¹⁸ ein gerad- oder verzweigtkettiger C₁- bis C₁₈-Alkyl-, -Alkenyl-, - Alkinylrest oder ein Arylrest ist, oder für über P an die C(O)-Gruppe gebundenes T-U-V steht, wobei T und U gerad- oder verzweigtkettige C₁- bis C₁₈-Alkylen-, -Alkenylen-, -Alkinylengruppen, alicyclische C₃- bis C₁₂-Gruppen oder Arylgruppen darstellen und gleich oder verschieden sind, und V ein gerad- oder verzweigtkettiger C₁- bis C₁₈-Alkyl-, - Alkenyl- oder -Alkinyl- oder ein Arylrest ist oder
R¹⁸ eine der vorerwähnten Bedeutungen hat und zusätzlich mit einer oder mehreren Gruppen NR¹⁹R²⁰ oder einer oder mehreren Gruppen SOₓR²¹ substituiert ist, wobei x = 0,1 oder 2 und R¹⁹, R²⁰ und R²¹ jeweils Wasserstoff oder über T an N, S gebundenes T-U-V mit der vorerwähnten Bedeutung sind, mit der Maßgabe, daß außerdem die physiologisch verträglichen Additionssalze mit anorganischen und organischen Säuren einbezogen sind,
und das Gestagen der nachstehenden Formel.

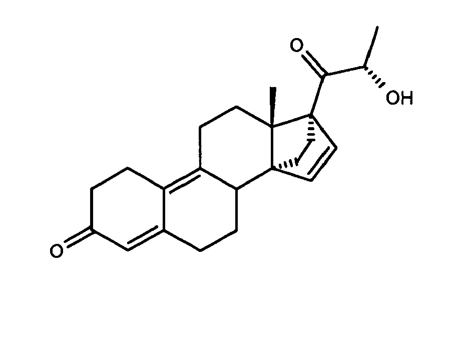

2. Zusammensetzung nach Anspruch 1, **dadurch gekennzeichnet, daß** das 11β-Halogensteroid der allgemeinen Formel I die Verbindung 11β-Fluor-17β-hydroxy-7α-methyl-estr-4-en-3-on ist.

3. Pharmazeutische Zusammensetzung, enthaltend eine Zusammensetzung gemäß Anspruch 1 sowie einen pharmazeutisch verträglichen Träger und/oder Hilfsstoffe.

4. Pharmazeutische Zusammensetzung nach Anspruch 3, **dadurch gekennzeichnet, daß** das 11 β-Halogensteroid der allgemeinen Formel I die Verbindung 11 β-Fluor-17β-hydroxy-7α-methyl-estr-4-en-3-on ist.

5. Männliches Kontrazeptivum, enthaltend eine pharmazeutische Zusammensetzung gemäß Anspruch 3.

6. Männliches Kontrazeptivum, enthaltend eine pharmazeutische Zusammensetzung gemäß Anspruch 4.

7. Männliches Kontrazeptivum nach Anspruch 5 oder 6, **dadurch gekennzeichnet, daß** die androgene Verbindung der allgemeinen Formel I darin mittels eines Implantats kontinuierlich über eine längere Zeitdauer verabreicht wird.

8. Männliches Kontrazeptivum nach Anspruch 5 oder 6, **dadurch gekennzeichnet, daß** die androgene Verbindung der allgemeinen Formel I darin oral verabreicht wird.

9. Männliches Kontrazeptivum nach Anspruch 5 oder 6, **dadurch gekennzeichnet, daß** die androgene Verbindung darin mittels eines transdermalen Systems über eine längere Zeitdauer verabreicht wird.

10. Männliches Kontrazeptivum nach einem der Ansprüche 5-9, **dadurch gekennzeichnet, daß** das Gestagen darin mittels eines Implantats über eine längere Zeitdauer kontinuierlich verabreicht wird.

11. Männliches Kontrazeptivum nach einem der Ansprüche 5-9, **dadurch gekennzeichnet, daß** das Gestagen in einem Transdermalsystem formuliert ist.

12. Männliches Kontrazeptivum nach einem der Ansprüche 5-9, **dadurch gekennzeichnet, daß** das Gestagen oral verabreicht wird.

## Claims

1. Composition containing an androgenic 11β-halogen steroid selected from the group of compounds of general formula I 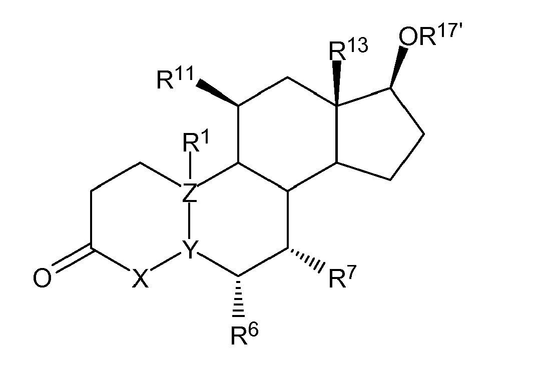 in which
X-Y-Z represents a group with one of the two structures CH=C-C or CH₂-C=C,
R¹ can be in α-position and β-position and stands for hydrogen, R or P-Q-R that is bonded via P to the basic ring structure, wherein P and Q represent straight-chain or branched-chain C₁- to C₈-alkylene, -alkenylene, or -alkinylene groups or their fluorinated derivatives and can be the same or different, and wherein R represents a CH₃ or CF₃ radical, provided that no substituent R¹ is present on Z if X-Y-Z represents the group CH₂-C=C,
R⁶ is a hydrogen atom or can have the meanings that are indicated under R⁷,
R⁷ stands for R or P-Q-R that is bonded via P to the basic ring structure, wherein these groups have the previously mentioned meanings,
R¹¹ represents a halogen,
R¹³ is methyl or ethyl, and
R^{17'} is hydrogen or stands for C(O)-R¹⁸, wherein
R¹⁸ is a straight-chain or branched-chain C₁- to C₁₈-alkyl, -alkenyl, or -alkinyl radical or an aryl radical, or stands for T-U-V that is bonded via P to the C(O) group, wherein T and U represent straight-chain or branched-chain C₁- to C₁₈-alkylene, -alkenylene, -alkinylene groups, alicyclic C₃- to C₁₂ groups or aryl groups and are the same or different, and V is a straight-chain or branched-chain C₁- to C₁₈-alkyl-, -alkenyl- or -alkinyl radical or an aryl radical, or
R¹⁸ has one of the previously mentioned meanings and in addition is substituted with one or more groups NR¹⁹R²⁰ or one or more groups SOₓR²¹, wherein x=0, 1 or 2, and R¹⁹, R²⁰ and R²¹ in each case are hydrogen or T-U-V that is bonded via T to N, S with the previously mentioned meaning, provided that, in addition, the physiologically compatible addition salts with inorganic and organic acids are included,
and the gestagen of the formula below.

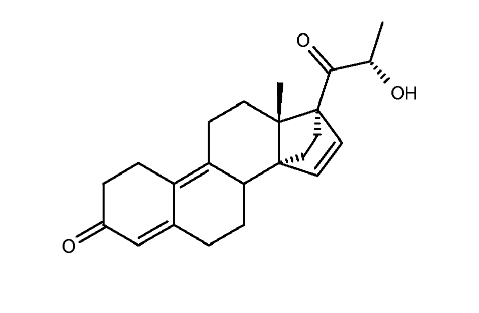

2. Composition according to Claim 1, **characterized in that** the 11β-halogen steroid of general formula I is the compound 11β-fluoro-17β-hydroxy-7α-methyl-estr-4-en-3-one.

3. Pharmaceutical composition containing a composition according to claim 1 as well as a pharmaceutically compatible vehicle and/or adjuvants.

4. Pharmaceutical composition according to claim 3, **characterized in that** the 11β-halogen steroid of general formula I is the compound 11β-fluoro-17β-hydroxy-7α-methyl-estr-4-en-3-one.

5. Male contraceptive agent containing a pharmaceutical composition according to Claim 3.

6. Male contraceptive agent containing a pharmaceutical composition according to claim 4.

7. Male contraceptive agent according to claim 5 or 6, **characterized in that** the androgenic compound of general formula I therein is administered by means of an implant continuosly over an extended period.

8. Male contraceptive agent according to claim 5 or 6, **characterized in that** the androgenic compound of general formula I therein is administered orally.

9. Male contraceptive agent according to claim 5 or 6, **characterized in that** the androgenic compound therein is administered by means of a transdermal system over an extended period.

10. Male contraceptive agent according to any of Claims 5-9, **characterized in that** the gestagen therein is administered by means of an implant continuosly over an extended period.

11. Male contraceptive agent according to any of claims 5-9, **characterized in that** the gestagen is formulated in a transdermal system.

12. Male contraceptive agent according to any of claims 5-9, **characterized in that** the gestagen is administered orally.

## Revendications

1. Composition contenant un 11β-halogénostéroïde androgène, choisi dans le groupe des composés de formule générale I 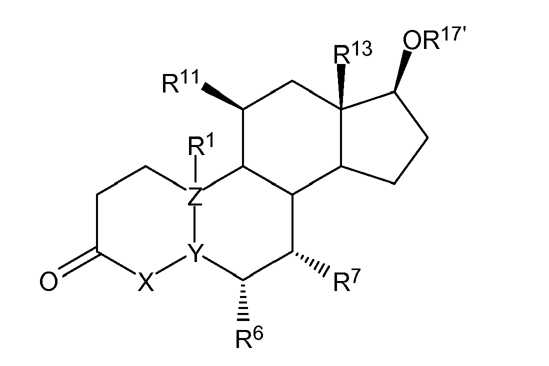 dans laquelle
X-Y-Z représente un groupe ayant l'une des deux structures CH=C-C ou CH₂-C=C
R¹ peut être en position α ou β et représente un atome d'hydrogène, R ou P-Q-R lié à la structure cyclique de base par P, P et Q représentant des groupes alkylène, alcénylène, alcynylène en C₁-C₈ à chaîne droite ou ramifiée et leurs dérivés fluorés et pouvant être identiques ou différents et R représentant un radical CH₃ ou CF₃, étant entendu qu'aucun substituant R¹ n'est présent sur Z lorsque X-Y-Z représente le groupe CH₂-C=C,
R⁶ est un atome d'hydrogène ou peut avoir les significations indiquées sous R⁷,
R⁷ représente R ou P-Q-R lié à la structure cyclique de base par P, ces groupes ayant les significations susmentionnées,
R¹¹ représente un atome d'halogène,
R¹³ est le groupe méthyle ou éthyle et
R^{17'} représente un atome d'hydrogène ou C(O)-R¹⁸, R¹⁸ étant un radical alkyle, alcényle, alcynyle en C₁-C₁₈ à chaîne droite ou ramifiée, ou représente T-U-V lié par P au groupe C(O), T et U représentant des groupes alkylène, alcénylène, alcynylène en C₁-C₁₈ à chaîne droite ou ramifiée, des groupes alicycliques en C₃-C₁₂ ou des groupes aryle et étant identiques ou différents, et V étant un radical alkyle, alcényle ou alcynyle en C₁-C₁₈ à chaîne droite ou ramifiée ou un radical aryle ou
R¹⁸ ayant l'une des significations susmentionnées et étant en outre substitué par un ou plusieurs groupes NR¹⁹R²⁰ ou un ou plusieurs groupes SOₓR²¹, où x = 0, 1 ou 2 et R¹⁹, R²⁰ et R²¹ représentent chacun un atome d'hydrogène ou T-U-V ayant la signification susmentionnée, lié par T à N, S, étant entendu que les sels d'addition avec des acides organiques ou minéraux, physiologiquement acceptables, sont en outre inclus,
et le progestatif de formule suivante

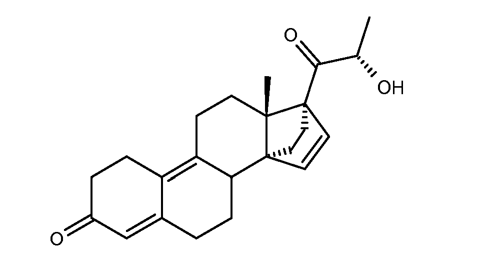

2. Composition selon la revendication 1, **caractérisée en ce que** le 11β-halogénostéroïde de formule générale I est le composé 11β-fluoro-17β-hydroxy-7α-méthyl-estr-4-én-3-one.

3. Composition pharmaceutique contenant une composition selon la revendication 1 ainsi qu'un véhicule pharmaceutiquement acceptable et/ou des adjuvants

4. Composition pharmaceutique selon la revendication 3, **caractérisée en ce que** le 11β-halogénostéroïde de formule générale I est le composé 11β-fluoro-17β-hydroxy-7α-méthyl-estr-4-én-3-one.

5. Contraceptif masculin contenant une composition pharmaceutique selon la revendication 3.

6. Contraceptif masculin contenant une composition pharmaceutique selon la revendication 4.

7. Contraceptif masculin selon la revendication 5 ou 6, **caractérisé en ce que** le composé androgène de formule générale I contenu dans celui-ci est administré au moyen d'un implant en continu pendant une relativement longue durée.

8. Contraceptif masculin selon la revendication 5 ou 6, **caractérisé en ce que** le composé androgène de formule générale I contenu dans celui-ci est administré par voie orale.

9. Contraceptif masculin selon la revendication 5 ou 6, **caractérisé en ce que** le composé androgène contenu dans celui-ci est administré pendant une relativement longue durée au moyen d'un système transdermique.

10. Contraceptif masculin selon l'une quelconque des revendications 5 à 9, **caractérisé en ce que** le progestatif contenu dans celui-ci est administré au moyen d'un implant en continu pendant une relativement longue durée.

11. Contraceptif masculin selon l'une quelconque des revendications 5 à 9, **caractérisé en ce que** le progestatif est formulé dans un système transdermique.

12. Contraceptif masculin selon l'une quelconque des revendications 5 à 9, **caractérisé en ce que** le progestatif est administré par voie orale.
